# EUROPEAN PATENT SPECIFICATION

(11) **EP 0 401 979 B1**
(45) Date of publication and mention of the grant of the patent: **04.09.1996**
(21) Application number: 90304996.3
(22) Date of filing: 09.05.1990
(51) Int. Cl.: C12N 15/32, A01N 63/02, C12N 1/20, C12P 21/02, C07K 14/00

(54) **Novel bacillus thuringiensis isolates active against lepidopteran pests, and genes encoding novel lepidopteran-active toxins**
Bacillus thuringiensis, der aktiv ist gegen Lepidoptera, und Gene, die Toxine gegen Lepidoptera kodieren
Souches de bacillus thuringiensis actives contre les lépidoptères nuisibles, et gènes codant pour des toxines actives contre les lépidoptères

(30) Priority: 18.05.1989 US 353860; 14.12.1989 US 451389
(43) Date of publication of application: 12.12.1990
(60) Divisional application: 95203119.3
(73) Proprietor: MYCOGEN CORPORATION, San Diego, California 92121 (US)
(72) Inventor: Payne, Jewel, San Diego, CA 92126 (US); Sick, August J., Oceanside, CA 92056 (US)
(74) Representative: Perry, Robert Edward

(56) References cited:
- EP-A- 0 063 949
- JOURNAL OF BIOLOGICAL CHEMISTRY, vol. 260, no. 10, 25 May 1985, American Society of Biological Chemists, Inc.; H.E. SCHNEPF et al., pp. 6264-6272
- NUCLEIC ACIDS RESEARCH, vol. 17, no. 1, 11 January 1989, Eynsham, Oxford (GB); L. MASSON et al., p. 446
- GENE, vol. 45, no. 3, 1986, Elsevier Science Publishers B.V., Amsterdam (NL); M.G. OBUKOWICZ et al., pp. 327-331

## Description

### Background of the Invention

The most widely used microbial pesticides are derived from the bacterium Bacillus thuringiensis. This bacterial agent is used to control a wide range of leaf-eating caterpillars and beetles, as well as mosquitos. Bacillus thuringiensis produces a proteinaceous parasporal body or crystal which is toxic upon ingestion by a susceptible insect host. For example, B. thuringiensis subsp. kurstaki HD-1 produces a crystal inclusion consisting of a biotoxin called a delta toxin which is toxic to the larvae of a number of lepidopteran insects. The cloning, sequencing, and expression of this B.t. crystal protein gene in Escherichia coli have been described by Schnepf et al [1981] Proc. Natl. Acad. Sci. USA 78:2893-2897. US-A-4,448,885 and US-A-4,467,036 both disclose the expression of B.t. crystal protein in E. coli.

### Brief Summary of the Invention

The subject invention concerns a novel Bacillus thuringiensis isolate designated PS81RR1 which has activity against all lepidopteran pests tested.

Also disclosed and claimed is a novel toxin gene which expresses a toxin toxic to lepidopteran insects. This toxin gene can be transferred to suitable hosts via a plasmid vector.

Specifically, the invention comprises a novel B.t. isolate denoted B.t. PS81RR1, mutants thereof, and a novel delta endotoxin gene derived from this B.t. isolate which encodes a protein which is active against lepidopteran pests. More specifically, the gene in B.t. PS81RR1 encodes a 133,367 dalton endotoxin.

### Detailed Disclosure of the Invention

The novel toxin gene of the subject invention was obtained from a novel lepidopteran-active B. thuringiensis (B.t.) isolate designated PS81RR1.

### Characteristics of B.t. PS81RR1

Colony morphology -- Large colony, dull surface, typical B.t.
Vegetative cell morphology -- typical B.t.
Flagellar serotype - 7, aizawai.
Intracellular inclusions -- sporulating cells produce a bipyramidal crystal.
Plasmid preparations -- agarose gel electrophoresis of plasmid preparations distinguishes PS81RR1 from B.t. HD-1 and other B.t. isolates.
Alkali-soluble proteins -- B.t. PS81RR1 produces a 133,367 dalton protein;
Unique toxin -- the 133,367 dalton toxin is different from any previously identified.
Activity -- B.t. PS81RR1 kills all Lepidoptera tested (Trichoplusia ni, Spodoptera exigua, and Plutella xylostella).
Bioassay procedures:
   Spodoptera exigua--dilutions are prepared of a spore and crystal pellet, mixed with USDA Insect Diet (Technical Bulletin 1528, U.S. Department of Agriculture) and poured into small plastic trays. Neonate Spodoptera exiqua larvae are placed on the diet mixture and held at 25°C. Mortality is recorded after six days.
   Other insects -- dilutions and diet are prepared in the same manner as for the Spodoptera exigua bioassay. Fourth instar larvae are used, and mortality is recorded after eight days.

B. thuringiensis PS81RR1, NRRL B-18458, and mutants thereof, can be cultured using standard known media and fermentation techniques. Upon completion of the fermentation cycle, the bacteria can be harvested by first separating the B.t. spores and crystals from the fermentation broth by means well known in the art. The recovered B.t. spores and crystals can be formulated into a wettable powder, a liquid concentrate, granules or other formulations by the addition of surfactants, dispersants, inert carriers and other components to facilitate handling and application for particular target pests. The formulation and application procedures are all well known in the art and are used with commercial strains of B. thuringiensis (HD-1) active against Lepidoptera, e.g., caterpillars. B.t. PS81RR1, and mutants thereof, can be used to control lepidopteran pests.

A subculture of B.t. PS81RR1 and the E. coli host harboring the toxin gene of the invention, were deposited in the permanent collection of the Northern Research Laboratory, U.S. Department of Agriculture, Peoria, Illinois, USA. The accession numbers and deposit dates are as follows:

| Subculture | Accession Number | Deposit Date |
|---|---|---|
| B.t. PS81RR1 | NRRL B-18458 | March 14, 1989 |
| E. coli (NM522) (pMYC390) | NRRL B-18449 | February 24, 1989 |

The toxin genes of the subject invention can be introduced into a wide variety of microbial hosts. Expression of the toxin gene results, directly or indirectly, in the intracellular production and maintenance of the pesticide. With suitable hosts, e.g., Pseudomonas, the microbes can be applied to the situs of lepidopteran insects where they will proliferate and be ingested by the insects. The result is a control of the unwanted insects. Alternatively, the microbe hosting the toxin gene can be treated under conditions that prolong the activity of the toxin produced in the cell. The treated cell then can be applied to the environment of target pest(s). The resulting product retains the toxicity of the B.t. toxin.

Where the B.t. toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera Bacillus, Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, and Alcaligenes; fungi, particularly yeast, e.g., genera Saccharomyces, Cryptococcus, Kluyveromyces, Sporobolomyces, Rhodotorula, and Aureobasidium. Of particular interest are such phytosphere bacterial species as Pseudomonas syringae. Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus, and Azotobacter vinlandii; and phytosphere yeast species such as Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae, and Aureobasidium pollulans. Of particular interest are the pigmented microorganisms.

A wide variety of ways are available for introducing a B.t. gene expressing a toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger RNA, particularly by using an active promoter, as well as by employing sequences, which enhance the stability of the messenger RNA. The transcriptional and translational termination region will involve stop codon(s), a terminator region, and optionally, a polyadenylation signal. A hydrophobic "leader" sequence may be employed at the amino terminus of the translated polypeptide sequence in order to promote secretion of the protein across the inner membrane.

In the direction of transcription, namely in the 5′ to 3′ direction of the coding or sense sequence, the construct will involve the transcriptional regulatory region, if any, and the promoter, where the regulatory region may be either 5′ or 3′ of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototropy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the Tac promoter, the naturally-occurring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present. The transformants then can be tested for pesticidal activity.

Suitable host cells, where the pesticide-containing cells will be treated to prolong the activity of the toxin in the cell when the then treated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae, Actinomycetales, and Nitrobacteraceae. Among eukaryotes are fungi, such as Phycomycetes and Ascomycetes, which includes yeast, such as Saccharomyces and Schizosaccharomyces; and Basidiomycetes yeast, such as Rhodotorula, Aureobasidium, Sporobolomyces, and the like.

Characteristics of particular interest in selecting a host cell for purposes of production include ease of introducing the B.t. gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., Streptomyces sp., and the like. Specific organisms include Pseudomonas aeruginosa, Pseudomonas fluorescens, Saccharomyces cerevisiae, Bacillus thuringiensis, Escherichia coli, Bacillus subtilis, Streptomyces lividans and the like.

The cell will usually be intact and be substantially in the proliferative form when treated, rather than in a spore form, although in some instances spores may be employed.

Treatment of the microbial cell, e.g., a microbe containing the B.t. toxin gene, can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Lugol iodine, Bouin's fixative, and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. Examples of physical means are short wavelength radiation such as gamma-radiation and X-radiation, freezing, UV irradiation, lyophilization, and the like.

The cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the B.t. insecticidal gene may be grown in any convenient nutrient medium, where the DNA construct provides a selective advantage, providing for a selective medium so that substantially all or all of the cells retain the B.t. gene. These cells may then be harvested in accordance with conventional ways. Alternatively, the cells can be treated prior to harvesting.

The B.t. cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the lepidopteran pest(s), e.g., plants, soil or water, by spraying, dusting, sprinkling, or the like.

Mutants of PS81RR1 can be made by procedures well known in the art. For example, an asporogenous mutant can be obtained through ethylmethane sulfonate (EMS) mutagenesis of PS81RR1. The mutants can be made using ultraviolet light and nitrosoguanidine by procedures well known in the art.

A smaller percentage of the asporogenous mutants will remain intact and not lyse for extended fermentation periods; these strains are designated lysis minus (-). Lysis minus strains can be identified by screening asporogenous mutants in shake flask media and selecting those mutants that are still intact and contain toxin crystals at the end of the fermentation. Lysis minus strains are suitable for a cell fixation process that will yield a protected, encapsulated toxin protein.

To prepare a phage resistant variant of said asporogenous mutant, an aliquot of the phage lysate is spread onto nutrient agar and allowed to dry. An aliquot of the phage sensitive bacterial strain is then plated directly over the dried lysate and allowed to dry. The plates are incubated at 30°C. The plates are incubated for 2 days and, at that time, numerous colonies could be seen growing on the agar. Some of these colonies are picked and subcultured onto nutrient agar plates. These apparent resistant cultures are tested for resistance by cross streaking with the phage lysate. A line of the phage lysate is streaked on the plate and allowed to dry. The presumptive resistant cultures are then streaked across the phage line. Resistant bacterial cultures show no lysis anywhere in the streak across the phage line after overnight incubation at 30°C. The resistance to phage is then reconfirmed by plating a lawn of the resistant culture onto a nutrient agar plate. The sensitive strain is also plated in the same manner to serve as the positive control. After drying, a drop of the phage lysate is plated in the center of the plate and allowed to dry. Resistant cultures showed no lysis in the area where the phage lysate has been placed after incubation at 30°C for 24 hours.

Following are examples which illustrate procedures, including the best mode, for practicing the invention. These examples should not be construed as limiting. All percentages are by weight and all solvent mixture proportions are by volume unless otherwise noted.

### Example 1 - Culturing B.t. PS81RR1

A subculture of PS81RR1, or mutants thereof, can be used to inoculate the following medium, a peptone, glucose, salts medium.

| | |
|---|---|
| Bacto Peptone | 7.5 g/l |
| Glucose | 1.0 g/l |
| KH₂PO₄ | 3.4 g/l |
| K₂HPO₄ | 4.35 g/l |
| Salt Solution | 5.0 ml/l |
| CaCl₂ Solution | 5.0 ml/l |

| Salts Solution (100 ml) | |
|---|---|
| MgSO₄.7H₂O | 2.46 g |
| MnSO₄.H₂O | 0.04 g |
| ZnSO₄.7H₂O | 0.28 g |
| FeSO₄.7H₂O | 0.40 g |

| CaCl₂ Solution (100 ml) | |
|---|---|
| CaCl₂.2H₂O | 3.66 g |
| pH 7.2 | |

The salts solution and CaCl₂ solution are filter-sterilized and added to the autoclaved and cooked broth at the time of inoculation. Flasks are incubated at 30°C on a rotary shaker at 200 rpm for 64 hr.

The above procedure can be readily scaled up to large fermentors by procedures well known in the art.

The B.t. spores and/or crystals, obtained in the above fermentation, can be isolated by procedures well known in the art. A frequently-used procedure is to subject the harvested fermentation broth to separation techniques, e.g., centrifugation.

### Example 2 - Cloning of Novel Toxin Gene From Isolate PSB1RR1 and Transformation into Escherichia coli

Total cellular DNA was prepared from B.t. cells grown to a low optical density (OD₆₀₀ = 1.0). The cells were recovered by centrifugation and protoplasted in TES buffer (30 mM Tris-Cl, 10 mM ethylenediaminetetraacetic acid [EDTA], 50 mM NaCl, pH = 8.0) containing 20% sucrose and 50 mg/ml lysozyme. The protoplasts were lysed by addition of sodium dodecyl sulfate (SDS) to a final concentration of 4%. The cellular material was precipitated overnight at 4°C in 100 mM (final concentration) neutral potassium chloride. The supernate was extracted twice with phenol/chloroform (1:1). The DNA was precipitated with ethanol and purified by isopycnic banding on a cesium chloride gradient.

Total cellular DNA from PS81RR1 and B.t.k. HD-1 was digested with EcoRI and separated by electrophoresis on a 0.8% Agarose-TAE-buffered gel. A Southern blot of the gel was probed with the NsiI to NsiI fragment of the toxin gene contained in plasmid pM3,130-7 of NRRL B-18332 and the NsiI to KpnI fragment of the "4.5 Kb class" toxin gene (Kronstad and Whitely [1986] Gene USA 43:29-40). These two fragments were combined and used as the probe. Results show that hybridizing fragments of PS81RR1 are distinct from those of HD-1. Specifically, a 2.3 Kb hybridizing band in PS81RR1 was detected instead of the 3.8 Kb and 1.8 Kb hybridizing bands seen in HD-1.

Two hundred micrograms of PS81RR1 total cellular DNA was digested with EcoRI and separated by electrophoresis on a preparative 0.8% Agarose-TAE gel. The 2.2 Kb to 2.4 Kb region of the gel was cut out and the DNA from it was electroeluted and concentrated using an ELUTlP™-d (Schleicher and Schuell, Keene, NH) ion exchange column. The isolated EcoRI fragments were ligated to LAMBDA ZAP™ EcoRI arms (Stratagene Cloning Systems, La Jolla, CA) and packaged using Gigapak GOLD™ (Stratagene) extracts. The packaged recombinant phage were plated with E. coli strain BB4 (Stratagene) to give high plaque density. The plaques were screened by standard nucleic acid hybridization procedure with radiolabeled probe. The plaques that hybridized were purified and re-screened at a lower plaque density. The resulting purified phage were grown with R408 M13 helper phage (Stratagene) and the recombinant BlueScript™ (Stratagene) plasmid was automatically excised and packaged. The "phagemid" was re-infected in XL1-Blue E. coli cells (Stratagene) as part of the automatic excision process. The infected XL1-Blue cells were screened for ampicillin resistance and the resulting colonies were analyzed by standard miniprep procedure to find the desired plasmid. The plasmid, designated pM3,31-3, contains an approximate 2.3 Kb EcoRI insert and was sequenced using Stratagene's T7 and T3 primers plus a set of existing B.t. endotoxin oligonucleotide primers. About 600 bp of the toxin gene was sequenced, and data analysis comparing PS81RR1 to other cloned B.t. endotoxin genes showed that the PS81RR1 sequence was unique. A synthetic oligonucleotide (CGTGGATATGGTGAATCTTATG) was constructed to one of the regions in the PS81RR1 sequence that was least homologous relative to other existing B.t. endotoxin genes.

PS81RR1 total cellular DNA partially digested with Sau3A and fractionated by electrophoresis into a mixture of 9-23 Kb fragments on a 0.6% agarose-TAE gel was ligated into Lambda GEM™-11 (PROMEGA). The packaged phage at a high titer were plated on P2392 E. coli cells (Stratagene) and screened using the radiolabeled synthetic oligonucleotide (aforementioned) as a nucleic acid hybridization probe. Hybridizing plaques were rescreened at a lower plaque density. A single purified hybridizing plaque was used to infect P2392 E. coli cells in liquid culture for preparation of phage for DNA isolation. DNA was isolated by standard procedures. Preparative amounts of recombinant phage DNA were digested with SalI, to release the inserted DNA from lambda arms, and separated by electrophoresis on a 0.6% Agarose-TAE gel. The large fragments, electroeluted and concentrated as described above, were ligated to SalI-digested and dephosphorylated pUC19 (NEB). The ligation mixture was introduced by transformation into E. coli DH5(alpha) competent cells (BRL) and plated on LB agar containing ampicillin, isopropyl-(Beta)-D-thiogalactoside (IPTG) and 5-Bromo-4-Chloro-3-indolyl-(Beta)-D-galactoside (XGAL). White colonies (with insertions in the (Beta)-galactosidase gene of pUC19) were subjected to standard miniprep procedures to isolate the plasmid, designated pM1,RR1-A The full length toxin gene was sequenced by using oligonucleotide primers made to the "4.5 Kb class" toxin gene and by "walking" with primers made to the sequence of PS81RR1.

The plasmid pM1,RR1-A contains about 13 Kb of PS81RR1 DNA including the 3.540 Kb which encodes the 133,367 dalton endotoxin. The ORF of the PS81RR1 toxin gene was isolated from pM1,RR1-A on a 3.8 Kb NdeI fragment and ligated into the Bacillus shuttle vector pBC1ac. E. coli NM522 cells were transformed and the resulting colonies were analyzed by standard miniprep procedures to isolate plasmids that contained the correct insert. The desired plasmid, pMYC390, contains the coding sequence of the PS81RR1 toxin gene.

The above cloning procedures were conducted using standard procedures unless otherwise noted.

The various methods employed in the preparation of the plasmids and transformation of host organisms are well known in the art. Also, methods for the use of lambda bacteriophage as a cloning vehicle, i.e., the preparation of lambda DNA, in vitro packaging, and transfection of recombinant DNA, are well known in the art. These procedures are all described in Maniatis, T., Fritsch, E.F., and Sambrook, J. (1982) Molecular Cloning: A Laboratory Manual, Cold Spring Harbor Laboratory, New York. Thus, it is within the skill of those in the genetic engineering art to extract DNA from microbial cells, perform restriction enzyme digestions, electrophorese DNA fragments, tail and anneal plasmid and insert DNA, ligate DNA, transform cells, prepare plasmid DNA, electrophorese proteins, and sequence DNA.

The restriction enzymes disclosed herein can be purchased from Bethesda Research Laboratories, Gaithersburg, MD, New England Biolabs, Beverly, MA, or Boehringer-Mannheim, Indianapolis, IN. The enzymes are used according to the instructions provided by the supplier.

Plasmid pMYC386 containing the B.t. toxin genes, can be removed from the transformed host microbes by use of standard well-known procedures. For example, E. coli NRRL B-18449 can be subjected to cleared lysate isopycnic density gradient procedures, and the like, to recover pMYC386.

The novel gene coding for the novel insecticidal toxin, as disclosed herein, can be inserted into plant cells using the Ti plasmid from Agrobacter tumefaciens. Plant cells can then be caused to regenerate into plants (Zambryski, P., Joos, H., Gentello, C., Leemans, J., Van Montague, M. and Schell, J [1983] Cell 32:1033-1043). A particularly useful vector in this regard is pEND4K (Klee, H.J., Yanofsky, M.F. and Nester, E.W. [1985] Bio/Technology 3:637-642). This plasmid can replicate both in plant cells and in bacteria and has multiple cloning sites for passenger genes. The toxin gene, for example, can be inserted into the BamHI site of pEND4K, propagated in E. coli and transformed into appropriate plant cells.

The novel gene of the invention can be cloned into baculoviruses such as Autographa californica nuclear polyhedrosis virus (AcNPV). Plasmids can be constructed that contain the AcNPV genome cloned into a commercial cloning vector such as pUC8. The AcNPV genome is modified so that the coding region of the polyhedrin gene is removed and a unique cloning site for a passenger gene is placed directly behind the polyhedrin promoter. Examples of such vectors are pGP-B6874, described by Pennock et al. (Pennock, G.D., Shoemaker, C. and Miller, L.K [1984] Mol. Cell. Biol. 4:399-406), and pAC380, described by Smith et al. (Smith, G.E., Summers, M.D. and Fraser, M.J. [1983] Mol Cell. Biol. 3:2156-2165). The gene coding for the novel protein toxin of the invention can be modified with BamHI linkers at appropriate regions both upstream and downstream from the coding region and inserted into the passenger site of one of the AcNPV vectors.

The nucleotide sequence encoding the novel B.t. toxin gene and the deduced amino acid sequence are shown in SEQ ID No. 1, below.

It is well known in the art that the amino acid sequence of a protein is determined by the nucleotide sequence of the DNA. Because of the redundancy of the genetic code, i.e. more than one coding nucleotide triplet (codon) can be used for most of the amino-acids used to make proteins, different nucleotide sequences can code for a particular amino-acid.

The novel B.t. toxins can be prepared via any nucleotide sequence (equivalent to that shown) encoding the same amino-acid sequence; the present invention includes such equivalent nucleotide sequences.

It has been shown that proteins of identified structure and function may be constructed by changing the amino-acid sequence, if such changes do not alter the protein secondary structure; see Kaiser, E.T. and Kezdy, F.J. (1984) Science 223:249-255. The present invention includes mutants of the amino-acid sequences depicted herein which have an unaltered protein secondary structure or, if the structure is altered, the mutant has the biological activity retained to some degree.

### SEQ ID No. X1

SEQUENCE TYPE: Nucleotide with corresponding protein
SEQUENCE LENGTH: 1179 bases
STRANDEDNESS: single
MOLECULE TYPE: genomic DNA
ORIGINAL SOURCE ORGANISM: bacterium
PROPERTIES: endotoxin

ATG GAG ATA ATG AAT AAT CAG AAT CAA TGC GTT CCT TAT AAC TGT TTG AAT GAT CCG ACA
Met Glu Ile Met Asn Asn Gln Asn Gln Cys Val Pro Tyr Asn Cys Leu Asn Asp Pro Thr
                5                   10                  15                  20

ATT GAA ATA TTA GAA GGA GAA AGA ATA GAA ACT GGT TAC ACC CCA ATA GAT ATT TCC TTG
Ile Glu Ile Leu Glu Gly Glu Arg Ile Glu Thr Gly Tyr Thr Pro Ile Asp Ile Ser Leu
                25                  30                  35                  40

TCG CTA ACG CAA TTT CTG TTG AGT GAA TTT GTC CCA GGT GCT GGG TTT GTA TTA GGT TTA
Ser Leu Thr Gln Phe Leu Leu Ser Glu Phe Val Pro Gly Ala Gly Phe Val Leu Gly Leu
                45                  50                  55                  60

ATT GAT TTA ATA TGG GGG TTT GTG GGT CCC TCT CAA TGG GAT GCA TTT CTT GTG CAA ATT
Ile Asp Leu Ile Trp Gly Phe Val Gly Pro Ser Gln Trp Asp Ala Phe Leu Val Gln Ile
                65                  70                  75                  80

GAA CAG TTA ATT AAC CAA AGA ATA GAG GAA TTC GCT AGG AAC CAA GCA ATT TCT AGA TTA
Glu Gln Leu Ile Asn Gln Arg Ile Glu Glu Phe Ala Arg Asn Gln Ala Ile Ser Arg Leu
                85                  90                  95                  100

GAA GGG CTA AGC AAC CTT TAT CAA ATT TAC GCA GAA GCT TTT AGA GAG TGG GAA GCA GAT
Glu Gly Leu Ser Asn Leu Tyr Gln Ile Tyr Ala Glu Ala Phe Arg Glu Trp Glu Ala Asp
                105                 110                 115                 120

CCT ACT AAT CCA GCA TTA ACA GAA GAG ATG CGT ATT CAG TTC AAT GAC ATG AAC AGT GCT
Pro Thr Asn Pro Ala Leu Thr Glu Glu Met Arg Ile Gln Phe Asn Asp Met Asn Ser Ala
                125                 130                 135                 140

CTT ACA ACC GCT ATT CCT CTT TTT ACA GTT CAA AAT TAT CAA GTA CCT CTT CTA TCA GTA
Leu Thr Thr Ala Ile Pro Leu Phe Thr Val Gln Asn Tyr Gln Val Pro Leu Leu Ser Val
                145                 150                 155                 160

TAT GTT CAA GCT GCA AAT TTA CAT TTA TCG GTT TTG AGA GAT GTT TCA GTG TTT GGA CAA
Tyr Val Gln Ala Ala Asn Leu His Leu Ser Val Leu Arg Asp Val Ser Val Phe Gly Gln
                165                 170                 175                 180

CGT TGG GGA TTT GAT GTA GCA ACA ATC AAT AGT CGT TAT AAT GAT TTA ACT AGG CTT ATT
Arg Trp Gly Phe Asp Val Ala Thr Ile Asn Ser Arg Tyr Asn Asp Leu Thr Arg Leu Ile
                185                 190                 195                 200

GGC ACC TAT ACA GAT TAT GCT GTA CGC TGG TAT AAT ACG GGA TTA GAA CGT GTA TGG GGA
Gly Thr Tyr Thr Asp Tyr Ala Val Arg Trp Tyr Asn Thr Gly Leu Glu Arg Val Trp Gly
                205                 210                 215                 220

CCG GAT TCT AGA GAT TGG GTA AGG TAT AAT CAA TTT AGA AGA GAG CTA ACA CTA ACT GTA
Pro Asp Ser Arg Asp Trp Val Arg Tyr Asn Gln Phe Arg Arg Glu Leu Thr Leu Thr Val
                225                 230                 235                 240

TTA GAT ATC GTT TCT CTG TTC CCG AAC TAT GAT AGT AGA ACG TAT CCA ATT CGA ACA GTT
Leu Asp Ile Val Ser Leu Phe Pro Asn Tyr Asp Ser Arg Thr Tyr Pro Ile Arg Thr Val
                245                 250                 255                 260

TCC CAA TTA ACT AGA GAA ATT TAT ACA AAC CCA GTA TTA GAA AAT TTT GAT GGT AGT TTT
Ser Gln Leu Thr Arg Glu Ile Tyr Thr Asn Pro Val Leu Glu Asn Phe Asp Gly Ser Phe
                265                 270                 275                 280

CGT GGA ATG GCT CAG AGA ATA GAA CAG AAT ATT AGG CAA CCA CAT CTT ATG GAT CTC CTT
Arg Gly Met Ala Gln Arg Ile Glu Gln Asn Ile Arg Gln Pro His Leu Met Asp Leu Leu
                285                 290                 295                 300

AAT AGT ATA ACC ATT TAT ACT GAT GTG CAT AGA GGC TTT AAT TAT TGG TCA GGA CAT CAA
Asn Ser Ile Thr Ile Tyr Thr Asp Val His Arg Gly Phe Asn Tyr Trp Ser Gly His Gln
                305                 310                 315                 320

ATA ACA GCT TCT CCT GTC GGT TTT GCG GGG CCA GAA TTT ACT TTT CCT AGA TAT GGA AAC
Ile Thr Ala Ser Pro Val Gly Phe Ala Gly Pro Glu Phe Thr Phe Pro Arg Tyr Gly Thr
                325                 330                 335                 340

ATG GGA AAT GCT GCT CCA CCC GTA CTG ATC TCA ACT ACT GGT TTG GGG ATT TTT AGA ACA
Met Gly Asn Ala Ala Pro Pro Val Leu Ile Ser Thr Thr Gly Leu Gly Ile Phe Arg Thr
                345                 350                 355                 360

TTA TCT TCA CCT CTT TAC AGA AGA ATT ATA CTT GGT TCA GGC CCA AAT AAT CAG AAC CTG
Leu Ser Ser Pro Leu Tyr Arg Arg Ile Ile Leu Gly Ser Gly Pro Asn Asn Gln Asn Leu
                365                 370                 375                 380

TTT GTC CTT GAT GGA ACG GAA TTT TCT TTT GCC TCC CTA ACA GCC GAT TTA CCT TCT ACT
Phe Val Leu Asp Gly Thr Glu Phe Ser Phe Ala Ser Leu Thr Ala Asp Leu Pro Ser Thr
                385                 390                 395                 400

ATA TAC AGA CAA AGG GGA ACG GTC GAT TCA CTA GAT GTA ATA CCG CCA CAG GAT AAT AGT
Ile Tyr Arg Gln Arg Gly Thr Val Asp Ser Leu Asp Val Ile Pro Pro Gln Asp Asn Ser
                405                 410                 415                 420

GTG CCA GCA CGT GCG GGA TTT AGT CAT CGA TTA AGT CAT GTT ACA ATG CTG AGC CAA GCA
Val Pro Ala Arg Ala Gly Phe Ser His Arg Leu Ser His Val Thr Met Leu Ser Gln Ala
                425                 430                 435                 440

GCT GGA GCA GTT TAC ACC TTG AGA GCT CCA ACG TTT TCT TGG CGA CAT CGT AGT GCT GAA
Ala Gly Ala Val Tyr Thr Leu Arg Ala Pro Thr Phe Ser Trp Arg His Arg Ser Ala Glu
                445                 450                 455                 460

TTC TCT AAC CTA ATT CCT TCA TCA CAA ATC ACA CAG ATA CCT TTA ACA AAG TCT ATT AAT
Phe Ser Asn Leu Ile Pro Ser Ser Gln Ile Thr Gln Ile Pro Leu Thr Lys Ser Ile Asn
                465                 470                 475                 480

CTT GGC TCT GGG ACC TCT GTT GTT AAA GGA CCA GGA TTT ACA GGA GGA GAT ATT CTT CGA
Leu Gly Ser Gly Thr Ser Val Val Lys Gly Pro Gly Phe Thr Gly Gly Asp Ile Leu Arg
                485                 490                 495                 500

AGA ACT TCA CCT GGC CAG ATT TCA ACC TTA AGA GTG ACT ATT ACT GCA CCA TTA TCA CAA
Arg Thr Ser Pro Gly Gln Ile Ser Thr Leu Arg Val Thr Ile Thr Ala Pro Leu Ser Gln
                505                 510                 515                 520

AGA TAT CGC GTA AGA ATT CGC TAC GCT TCT ACT ACA AAT TTA CAA TTC CAT ACA TCA ATT
Arg Tyr Arg Val Arg Ile Arg Tyr Ala Ser Thr Thr Asn Leu Gln Phe His Thr Ser Ile
                525                 530                 535                 540

GAC GGA AGA CCT ATT AAT CAG GGG AAT TTT TCA GCA ACT ATG AGT AGT GGG GGT AAT TTA
Asp Gly Arg Pro Ile Asn Gln Gly Asn Phe Ser Ala Thr Met Ser Ser Gly Gly Asn Leu
                545                 550                 555                 560

CAG TCC GGA AGC TTT AGG ACT GCA GGT TTT ACT ACT CCG TTT AAC TTT TCA AAT GGA TCA
Gln Ser Gly Ser Phe Arg Thr Ala Gly Phe Thr Thr Pro Phe Asn Phe Ser Asn Gly Ser
                565                 570                 575                 580

AGT ATA TTT ACG TTA AGT GCT CAT GTC TTC AAT TCA GGC AAT GAA GTT TAT ATA GAT CGA
Ser Ile Phe Thr Leu Ser Ala His Val Phe Asn Ser Gly Asn Glu Val Tyr Ile Asp Arg
                585                 590                 595                 600

ATT GAA TTT GTT CCG GCA GAA GTA ACA TTT GAG GCG GAA TAT GAT TTA GAA AGA GCG CAA
Ile Glu Phe Val Pro Ala Glu Val Thr Phe Glu Ala Glu Tyr Asp Leu Glu Arg Ala Gln
                605                 610                 615                 620

GAG GCG GTG AAT GCT CTG TTT ACT TCT TCC AAT CAA CTA GGA TTA AAA ACA AAT GTG ACG
Glu Ala Val Asn Ala Leu Phe Thr Ser Ser Asn Gln Leu Gly Leu Lys Thr Asn Val Thr
                625                 630                 635                 640

GAC TAT CAT ATT GAT CAA GTG TCC AAT CTA GTC GAA TGT TTA TCC GGT GAA TTC TGT CTG
Asp Tyr His Ile Asp Gln Val Ser Asn Leu Val Glu Cys Leu Ser Gly Glu Phe Cys Leu
                645                 650                 655                 660

GAT GAA AAG AGA GAA TTG TCC GAG AAA GTC AAA CAT GCG AAG CGA CTC AGT GAT GAG CGG
Asp Glu Lys Arg Glu Leu Ser Glu Lys Val Lys His Ala Lys Arg Leu Ser Asp Glu Arg
                665                 670                 675                 680

AAT TTA CTT CAA GAC CCA AAC TTC AGA GGC ATC AAT AGA CAA CCA GAC CGT GGC TGG AGA
Asn Leu Leu Gln Asp Pro Asn Phe Arg Gly Ile Asn Arg Gln Pro Asp Arg Gly Trp Arg
                685                 690                 695                 700

GGC AGT ACG GAT ATT ACC ATC CAA GGA GGA GAT GAC GTA TTC AAA GAG AAT TAC GTC ACA
Gly Ser Thr Asp Ile Thr Ile Gln Gly Gly Asp Asp Val Phe Lys Glu Asn Tyr Val Thr
                705                 710                 715                 720

CTA CCG GGT ACC TTT AAT GAG TGT TAT CCT ACG TAT CTG TAT CAA AAA ATA GAT GAG TCG
Leu Pro Gly Thr Phe Asn Glu Cys Tyr Pro Thr Tyr Leu Tyr Gln Lys Ile Asp Glu Ser
                725                 730                 735                 740

AAA TTA AAA GCC TAT ACC CGT TAC CAA TTA AGA GGG TAC ATC GAG GAT AGT CAA GAC TTA
Lys Leu Lys Ala Tyr Thr Arg Tyr Gln Leu Arg Gly Tyr Ile Glu Asp Ser Gln Asp Leu
                745                 750                 755                 760

GAA ATC TAT TTA ATT CGC TAC AAT ACA AAA CAC GAA ACA GTA AAT GTG CCA GGT ACG GGT
Glu Ile Tyr Leu Ile Arg Tyr Asn Thr Lys His Glu Thr Val Asn Val Pro Gly Thr Gly
765 770 775 78[?]

TCC TTA TGG CCG CTT TCA GTC GAA AAT CCA ATT GGA AAG TGC GGA GAA CCA AAT CGA TGC
Ser Leu Trp Pro Leu Ser Val Glu Asn Pro Ile Gly Lys Cys Gly Glu Pro Asn Arg Cys
785 790 795 800

GCA CCA CAA CTT GAA TGG AAT CCT GAT CTA GAT TGT TCC TGC AGA GAC GGG GAA AAA TGT
Ala Pro Gln Leu Glu Trp Asn Pro Asp Leu Asp Cys Ser Cys Arg Asp Gly Glu Lys Cys
805 810 815 820

GCA CAT CAC TCC CAT CAT TTC TCC TTG GAC ATT GAT ATT GGA TGT ACA GAT TTA AAT GAG
Ala His His Ser His His Phe Ser Leu Asp Ile Asp Ile Gly Cys Thr Asp Leu Asn Glu
825 830 835 840

AAC TTA GGT GTA TGG GTG ATA TTC AAA ATT AAG ACG CAA GAT GGT CAC GCA AGA CTA GGT
Asn Leu Gly Val Trp Val Ile Phe Lys Ile Lys Thr Gln Asp Gly His Ala Arg Leu Gly
845 850 855 860

AAT CTA GAG TTT CTC GAA GAG AAA CCA TTA GTA GGC GAA TCG TTA GCA CGC GTG AAG AGA
Asn Leu Glu Phe Leu Glu Glu Lys Pro Leu Val Gly Glu Ser Leu Ala Arg Val Lys Arg
865 870 875 880

GCG GAG AAG AAG TGG AGA GAC AAA CGA GAG AAA TTG CAA GTG GAA ACA AAT ATC GTT TAT
Ala Glu Lys Lys Trp Arg Asp Lys Arg Glu Lys Leu Gln Val Glu Thr Asn Ile Val Tyr
885 890 895 900

AAA GAG GCA AAA GAA TCT GTA GAT GCT TTA TTT GTG AAC TCT CAA TAT GAT AGA TTA CAA
Lys Glu Ala Lys Glu Ser Val Asp Ala Leu Phe Val Asn Ser Gln Tyr Asp Arg Leu Gln
905 910 915 920

GCG GAT ACC GAC ATC GCG ATG ATT CAT GCG GCA GAT AAA CGC GTT CAT CGA ATT CGA GAA
Ala Asp Thr Asp Ile Ala Met Ile His Ala Ala Asp Lys Arg Val His Arg Ile Arg Glu
925 930 935 940

GCA TAT CTT CCA GAG TTA TCT GTA ATT CCG GGT GTC AAT GCG GGC ATT TTT GAA GAA TTA
Ala Tyr Leu Pro Glu Leu Ser Val Ile Pro Gly Val Asn Ala Gly Ile Phe Glu Glu Leu
945 950 955 960

GAG GGA CGT ATT TTC ACA GCC TAC TCT TTA TAT GAT GCG AGA AAT GTC ATT AAA AAT GGC
Glu Gly Arg Ile Phe Thr Ala Tyr Ser Leu Tyr Asp Ala Arg Asn Val Ile Lys Asn Gly
965 970 975 980

GAT TTC AAT AAT GGC TTA TCA TGC TGG AAC GTG AAA GGG CAT GTA GAT GTA GAA GAA CAA
Asp Phe Asn Asn Gly Leu Ser Cys Trp Asn Val Lys Gly His Val Asp Val Glu Glu Gln
985 990 995 1000

AAC AAC CAC CGT TCG GTT CTT GTT GTC CCG GAA TGG GAA GCA GAG GTG TCA CAA GAG GTT
Asn Asn His Arg Ser Val Leu Val Val Pro Glu Trp Glu Ala Glu Val Ser Gln Glu Val
1005 1010 1015 1020

CGT GTC TGT CCA GGT CGT GGC TAT ATC CTA CGT GTT ACA GCG TAC AAA GAG GGA TAT GGA
Arg Val Cys Pro Gly Arg Gly Tyr Ile Leu Arg Val Thr Ala Tyr Lys Glu Gly Tyr Gly
1025 1030 1035 1040

GAA GGT TGC GTA ACG ATT CAT GAG ATC GAA GAC AAT ACA GAC GAA CTG AAA TTC AGC AAC
Glu Gly Cys Val Thr Ile His Glu Ile Glu Asp Asn Thr Asp Glu Leu Lys Phe Ser Asn
1045 1050 1055 1060

TGT GTA GAA GAG GAA GTA TAT CCA AAC AAC ACG GTA ACG TGT AAT GAT TAT ACT GCA AAT
Cys Val Glu Glu Glu Val Tyr Pro Asn Asn Thr Val Thr Cys Asn Asp Tyr Thr Ala Asn
1065 1070 1075 1080

CAA GAA GAA TAC GGG GGT GCG TAC ACT TCT CGT AAT CGT GGA TAT GGT GAA TCT TAT GAA
Gln Glu Glu Tyr Gly Gly Ala Tyr Thr Ser Arg Asn Arg Gly Tyr Gly Glu Ser Tyr Glu
1085 1090 1095 1100

AGT AAT TCT TCC ATA CCA GCT GAG TAT GCG CCA GTT TAT GAG GAA GCA TAT ATA GAT GGA
Ser Asn Ser Ser Ile Pro Ala Glu Tyr Ala Pro Val Tyr Glu Glu Ala Tyr Ile Asp Gly
1105 1110 1115 1120

AGA AAA GAG AAT CCT TGT GAA TCT AAC AGA GGA TAT GGG GAT TAC ACG CCA CTA CCA GCT
Arg Lys Glu Asn Pro Cys Glu Ser Asn Arg Gly Tyr Gly Asp Tyr Thr Pro Leu Pro Ala
1125 1130 1135 1140

GGT TAT GTG ACA AAA GAA TTA GAG TAC TTC CCA GAA ACC GAT AAG GTA TGG ATT GAG ATC
Gly Tyr Val Thr Lys Glu Leu Glu Tyr Phe Pro Glu Thr Asp Lys Val Trp Ile Glu Ile
1145 1150 1155 1160

GGG GAA ACG GAA GGA ACA TTC ATC GTG GAT AGC GTG GAA TTA CTC CTT ATG GAG GAA
Gly Glu Thr Glu Gly Thr Phe Ile Val Asp Ser Val Glu Leu Leu Leu Met Glu Glu
1165 1170 1175

## Claims (Claims for the following Contracting State(s): AT, BE, CH, DE, DK, FR, GB, GR, IT, LI, LU, NL, SE)

1. Bacillus thuringiensis PS81RR1, as available under the Accession Number NRRLB-18458.

2. DNA encoding a Bacillus thuringiensis toxin having the amino-acid sequence shown in SEQ ID No. 1.

3. DNA according to claim 2, having the nucleotide sequence shown in SEQ ID No. 1.

4. A toxin having the amino-acid sequence shown in SEQ ID. No. 1.

5. A recombinant DNA transfer vector comprising DNA according to claim 2 or claim 3.

6. A prokaryotic or eukaryotic host into which a DNA transfer vector according to claim 5 has been transferred and replicated.

7. A microorganism capable of expressing a Bacillus thuringiensis toxin having the amino-acid sequence shown in SEQ ID No. 1.

8. A microorganism according to claim 7, which is a species of Pseudomonas, Azotobacter, Erwinia, Serratia, Klebsiella, Rhizobium, Rhodopseudomonas, Methylophilius, Agrobacterium, Acetobacter or Alcaliagenes; a prokaryote selected from Enterbacteriaceae, Bacillaceae, Rhizobiaceae, Spirillaceae, Lactobacillaceae, Pseudomonadaceae, Azotobacteraceae and Nitrobacteraceae; or a lower eukaryote selected from Phycomycetes, Ascomycetes and Basidiomycetes.

9. A microorganism according to claim 8, which is Pseudomonas fluorescens or Escherichia coli.

10. A microorganism according to claim 9, which is E. coli (NM522) (pMYC 390), as available under the Accession Number NRRL B-18449.

11. A microorganism according to claim 7, which is a pigmented bacterium, yeast or fungus.

12. A microorganism according to any of claims 7 to 11, which is pigmented and phylloplane-adherent.

13. Substantially intact cells of a unicellular microorganism according to any of claims 1 and 6 to 12, containing the toxin.

14. Cells according to claim 13, as obtained by treatment with iodine or other chemical or physical means to prolong the insecticidal activity in the environment.

15. A composition comprising a microorganism according to any of claims 1 and 6 to 12, in association with an insecticide carrier or with formulation ingredients to be applied as a seed coating.

16. A composition according to claim 15, wherein the microorganism is in the form of spores or crystals.

17. A composition according to claim 15 or claim 16, wherein the carrier comprises beetle phagostimulants or attractants.

18. A method for controlling a lepidopteran insect pest, which comprises contacting the pest or its environment with a microorganism according to any of claims 1 and 6 to 12.

19. A method according to claim 18, wherein administration is to the rhizosphere, to the phylloplane, or to a body of water.

20. A method according to claim 18, which comprises placing a bait granule comprising the microorganism, e.g. as spores or crystals, on or in the soil when planting seed of a plant upon which the pest is known to feed.

21. A method according to claim 20, wherein the bait granule is placed at the same time as corn seed is planted in the soil.

22. Plasmid pMYC 390, as available in a host according to claim 10.

## Claims (Claims for the following Contracting State(s): ES)

1. A method of transforming a prokaryotic or eukaryotic host, which comprises transferring thereinto a DNA transfer vector comprising DNA encoding a Bacillus thuringiensis toxin having the amino-acid sequence shown in SEQ ID No. 1.

2. A method according to claim 1, wherein the DNA has the nucleotide sequence shown in SEQ ID No. 1.

3. A method for controlling a lepidopteran insect pest, which comprises contacting the pest or its environment with Bacillus thuringiensis PS81RR1, as available under the accession number NRRL B-18458.

4. A method for controlling a lepidopteran insect pest, which comprises contacting the pest or its environment with a prokaryotic or eukaryotic host microorganism into which a DNA transfer vector as defined in claim 1 or claim 2 has been transferred and replicated.

5. A method for controlling a lepidopteran insect pest, which comprises contacting the pest or its environment with a microorganism capable of expressing a Bacillus thuringiensis toxin having the amino-acid sequence shown in SEQ ID No. 1.

6. A method according to claim 5, wherein the microorganism is a species of Pseudomonas, Azotobacter, Erwinia, Serratia, Klebsiella, Rhizobium, Rhodopseudomonas, Methylophilius, Agrobacterium, Acetobacter or Alcaligenes; a prokaryote selected from Enterbacteriaceae, Bacillaceae, Rhizobiaceae, Spirillaceae, Lactobacillaceae, Pseudomonadaceae, Azotobacteraceae and Nitrobacteraceae; or a lower eukaryote selected from Phycomycetes, Ascomycetes and Basidiomycetes.

7. A method according to claim 6, wherein the microorganism is Pseudomonas fluorescens or Escherichia coli.

8. A method according to claim 7, wherein the microorganism is E. coli (NM522) (pMYC 390), as available under the accession number NRRL B-18449.

9. A method according to claim 5, wherein the microorganism is a pigmented bacterium, yeast or fungus.

10. A method according to any of claims 5 to 9, wherein the microorganism is pigmented and phylloplane-adherent.

11. A method according any of claims 3 to 10, wherein administration is to the rhizosphere, to the phylloplane, or to a body of water.

12. A method according to any of claims 3 to 10, which comprises placing a bait granule comprising the microorganism, e.g. as spores or crystals, on or in the soil when planting seed of a plant upon which the pest is known to feed.

13. A method according to claim 12, wherein the bait granule is placed at the same time as corn seed is planted in the soil.

14. A process for producing a composition comprising a microorganism as defined in any of claims 3 to 10, which comprises formulating the microorganism with an insecticide carrier or with formulation ingredients to be applied as a seed coating.

15. A process according to claim 14, wherein the microorganism is in the form of spores or crystals.

16. A process according to claim 14 or claim 15, wherein the carrier comprises beetle phagostimulants or attractants.

## Patentansprüche (Patentansprüche für folgende(n) Vertragsstaat(en): AT, BE, CH, DE, DK, FR, GB, GR, IT, LI, LU, NL, SE)

1. Bacillus thuringiensis PS81RRI, wie erhältlich unter der Hinterlegungsnummer NRRL B-18458.

2. DNA, die für ein Bacillus thuringiensis-Toxin mit der in SEQ ID Nr.1 dargestellten Aminosäuresequenz kodiert.

3. DNA nach Anspruch 2, welche die in SEQ ID Nr.1 dargestellte Nukleotidsequenz aufweist.

4. Toxin, welches die in SEQ ID Nr.l dargestellte Aminosäuresequenz aufweist.

5. Rekombinanter DNA-Transfer-Vektor, der DNA nach Anspruch 2 oder Anspruch 3 umfaßt.

6. Prokaryotischer oder eukaryotischer Wirt, in den ein DNA-Transfer-Vektor nach Anspruch 5 transferiert und repliziert worden ist.

7. Mikroorganismus, der imstande ist, ein Bacillus thuringiensis-Toxin mit der in SEQ ID Nr.1 dargestellten Aminosäuresequenz zu exprimieren.

8. Mikroorganismus nach Anspruch 7, der eine Spezies ist von Pseudomonas, Azotobacter, Erwinia, Serratia, Klebsiella, Rhizobium, Rhodopseudomonas, Methylophilius, Agrobacterium, Acetobacter oder Alcaligenes; ein aus Enterobacteriaceae, Bacillaceae, Rhizobiaceae, Spirillaceae, Lactobacillaceae, Pseudomonadaceae, Azotobacteraceae und Nitrobacteraceae ausgewählter Prokaryot; oder ein aus Phycomycetes, Ascomycetes und Basidiomycetes ausgewählter niederer Eukaryot.

9. Mikroorganismus nach Anspruch 8, der Pseudomonas fluorescens oder Escherichia coli ist.

10. Mikroorganismus nach Anspruch 9, der E. coli (NM522) (pMYC 390) ist, wie erhältlich unter der Hinterlegungsnummer NRRL B-18449.

11. Mikroorganismus nach Anspruch 7, der ein pigmentiertes Bakterium, Hefe oder ein Pilz ist.

12. Mikroorganismus nach irgendeinem der Ansprüche 7 bis 11, der pigmentiert ist und Phyllooberflächen-adhärent.

13. Im wesentlichen intakte Zellen eines einzelligen Mikroorganismus nach irgendeinem der Ansprüche 1 und 6 bis 12, der das Toxin enthält.

14. Zellen nach Anspruch 13, wie erhalten durch Behandlung mit Iod oder anderen chemischen oder physikalischen Mitteln, um die insektizide Aktivität in der Umwelt zu verlängern.

15. Zusammensetzung, umfassend einen Mikroorganismus nach irgendeinem der Ansprüche 1 und 6 bis 12 in Verbindung mit einem Insektizid-Träger oder mit Formulierungsbestandteilen für die Anwendung als Samenüberzug.

16. Zusammensetzung nach Anspruch 15, worin der Mikroorganismus in Form von Sporen oder Kristallen vorliegt.

17. Zusammensetzung nach Anspruch 15 oder Anspruch 16, worin der Träger Phagostimulantien oder Lockstoffe für Insekten umfaßt.

18. Verfahren zur Kontrolle eines Lepidoptera-InsektenSchädlings, welches umfaßt, daß der Schädling oder dessen Umwelt mit einem Mikroorganismus nach irgendeinem der Ansprüche 1 und 6 bis 12 in Kontakt gebracht wird.

19. Verfahren nach Anspruch 18, worin die Ausbringung in der Rhizosphäre, der Phyllooberfläche oder einem Wasserkörper erfolgt.

20. Verfahren nach Anspruch 18, welches umfaßt, daß ein Köderkörnchen, welches den Mikroorganismus, z.B. als Sporen oder Kristalle, umfaßt, auf oder in dem Erdreich ausgebracht wird, wenn Samen einer Pflanze, von der sich der Schädling bekanntermaßen ernährt, gepflanzt wird.

21. Verfahren nach Anspruch 20, worin das Köderkörnchen gleichzeitig mit dem Einpflanzen von Getreidesamen in das Erdreich ausgebracht wird.

22. Plasmid pMYC 390, wie erhältlich in einem Wirt nach Anspruch 10.

## Patentansprüche (Patentansprüche für folgende(n) Vertragsstaat(en): ES)

1. Verfahren zur Transformation eines prokaryotischen oder eukaryotischen Wirts, welches umfaßt, daß in diesen ein DNA-Transfer-Vektor transferiert wird, der DNA umfaßt, die für ein Bacillus thuringiensis-Toxin mit der in SEQ ID Nr.1 dargestellten Aminosäuresequenz kodiert.

2. Verfahren nach Anspruch 1, worin die DNA die in SEQ ID Nr.l dargestellte Nukleotidsequenz aufweist.

3. Verfahren zur Kontrolle eines Lepidoptera-Insekten-Schädlings, welches umfaßt, daß der Schädling oder dessen Umwelt mit Bacillus thuringiensis PS81RRI, wie erhältlich unter der Hinterlegungsnummer NRRL B-18458, in Kontakt gebracht wird.

4. Verfahren zur Kontrolle eines Lepidoptera-Insekten-Schädlings, welches umfaßt, daß der Schädling oder dessen Umwelt mit einem prokaryotischen oder eukaryotischen Wirts-Mikroorganismus, in den ein DNA-Transfer-Vektor, wie in Anspruch 1 oder Anspruch 2 definiert, transferiert und repliziert worden ist, in Kontakt gebracht wird.

5. Verfahren zur Kontrolle eines Lepidoptera-Insekten-Schädlings, welches umfaßt, daß der Schädling oder dessen Umwelt mit einem Mikroorganismus in Kontakt gebracht wird, welcher imstande ist, ein Bacillus thuringiensis-Toxin mit der in SEQ ID Nr.1 dargestellten Aminosäuresequenz zu exprimieren.

6. Verfahren nach Anspruch 5, worin der Mikroorganismus eine Spezies ist von Pseudomonas, Azotobacter, Erwinia, Serratia, Klebsiella, Rhizobium, Rhodopseudomonas, Methylophilius, Agrobacterium, Acetobacter oder Alcaligenes; ein aus Enterobacteriaceae, Bacillaceae, Rhizobiaceae, Spirillaceae, Lactobacillaceae, Pseudomonadaceae, Azotobacteraceae und Nitrobacteraceae ausgewählter Prokaryot; oder ein aus Phycomycetes, Ascomycetes und Basidiomycetes ausgewählter niederer Eukaryot.

7. Verfahren nach Anspruch 6, worin der Mikroorganismus Pseudomonas fluorescens oder Escherichia coli ist.

8. Verfahren nach Anspruch 7, worin der Mikroorganismus E. coli (NM522) (pMYC 390) ist, wie erhältlich unter der Hinterlegungsnummer NRRL B-18449.

9. Verfahren nach Anspruch 5, worin der Mikroorganismus ein pigmentiertes Bakterium, Hefe oder ein Pilz ist.

10. Verfahren nach irgendeinem der Ansprüche 5 bis 9, worin der Mikroorganismus pigmentiert und Phyllooberflächen-adhärent ist.

11. Verfahren nach irgendeinem der Ansprüche 3 bis 10, worin die Ausbringung in der Rhizosphäre, der Phyllooberfläche oder einem Wasserkörper erfolgt.

12. Verfahren nach irgendeinem der Ansprüche 3 bis 10, welches umfaßt, daß ein Köderkörnchen, welches den Mikroorganismus, z.B. als Sporen oder Kristalle, umfaßt, auf oder in dem Erdreich ausgebracht wird, wenn Samen einer Pflanze, von der sich der Schädling bekanntermaßen ernährt, gepflanzt wird.

13. Verfahren nach Anspruch 12, worin das Köderkörnchen gleichzeitig mit dem Einpflanzen von Getreidesamen in das Erdreich ausgebracht wird.

14. Verfahren zur Herstellung einer Zusammensetzung, umfassend einen Mikroorganismus wie in irgendeinem der Ansprüche 3 bis 10 definiert, welches umfaßt, daß der Mikroorganismus mit einem Insektizid-Träger oder mit Formulierungsbestandteilen für die Anwendung als Samenüberzug formuliert wird.

15. Verfahren nach Anspruch 14, worin der Mikroorganismus in Form von Sporen oder Kristallen vorliegt.

16. Zusammensetzung nach Anspruch 14 oder Anspruch 15, worin der Träger Phagostimulantien oder Lockstoffe für Insekten umfaßt.

## Revendications (Revendications pour l'(les) Etat(s) contractant(s) suivant(s): AT, BE, CH, DE, DK, FR, GB, GR, IT, LI, LU, NL, SE)

1. Bacillus thuringiensis PS81RR1, disponible sous le numéro d'accession NRRLB-18458.

2. ADN codant pour une toxine de Bacillus thuringiensis ayant la séquence d'acides aminés présentée dans SEQ ID No. 1.

3. ADN selon la revendication 2, ayant la séquence nucléotidique présentée dans SEQ ID No. 1.

4. Toxine ayant la séquence d'acides aminés présentée dans SEQ ID No. 1.

5. Vecteur de transfert d'ADN recombinant comprenant de l'ADN conforme à la revendication 2 ou à la revendication 3.

6. Hôte procaryote ou eucaryote dans lequel un vecteur de transfert d'ADN conforme à la revendication 5 a été transféré et répliqué.

7. Micro-organisme capable d'exprimer une toxine de Bacillus thuringiensis ayant la séquence d'acides aminés présentée dans SEQ ID No. 1.

8. Micro-organisme selon la revendication 7, qui est une espèce de Pseudomonas, d'Azotobacter, d'Erwinia, de Serratia, de Klebsiella, de Rhizobium, de Rhodopseudomonas, de Méthylophilius, d'Agrobacterium, d'Acetobacter ou d'Alcaligenes ; procaryote choisi parmi les entérobactériacées, les bacillacées, les rhizobiacées, les spirillacées, les lactobacillacées, les pseudomonadacées, les azotobactéracées et les nitrobactéracées ; ou eucaryote inférieur choisi parmi les phycomycètes, les ascomycètes et les basidiomycètes.

9. Micro-organisme selon la revendication 8, qui est Pseudomonas fluorescens ou Escherichia coli.

10. Micro-organisme selon la revendication 9, qui est E. coli (NM522) (pMYC 390), disponible sous le numéro d'accès NRRL B-18449.

11. Micro-organisme selon la revendication 7, qui est une bactérie, une levure ou un champignon pigmenté.

12. Micro-organisme selon l'une quelconque des revendications 7 à 11, qui est pigmenté et adhère au phylloplan.

13. Cellules pratiquement intactes d'un micro-organisme unicellulaire selon l'une quelconque des revendications 1 et 6 à 12, contenant la toxine.

14. Cellules selon la revendication 13, obtenues par traitement avec de l'iode ou un autre moyen chimique ou physique permettant de prolonger l'activité insecticide dans l'environnement.

15. Composition comprenant un micro-organisme selon l'une quelconque des revendications 1 et 6 à 12, en association avec un véhicule pour insecticide ou avec des ingrédients de formulation, à appliquer sous forme de revêtement de semence.

16. Composition selon la revendication 15, dans laquelle le micro-organisme est sous forme de spores ou de cristaux.

17. Composition selon la revendication 15 ou la revendication 16, dans laquelle le véhicule comprend des phagostimulants ou des attractifs pour coléoptères.

18. Procédé de lutte contre un lépidoptère nuisible, qui comprend la mise en contact du nuisible ou de son milieu avec un micro-organisme selon l'une quelconque des revendications 1 et 6 à 12.

19. Procédé selon la revendication 18, dans lequel l'administration se fait sur la rhizosphère, sur le phylloplan ou sur un volume d'eau.

20. Procédé selon la revendication 18, qui comprend le fait de placer un granule appât contenant le micro-organisme, par exemple sous forme de spores ou de cristaux sur ou dans le sol au moment du semis de la graine d'une plante sur laquelle on sait que le nuisible se nourrit.

21. Procédé selon la revendication 20, dans lequel le granule appât est placé au moment même où le grain de céréale est semé dans le sol.

22. Plasmide pMYC 390, disponible dans un hôte selon la revendication 10.

## Revendications (Revendications pour l'(les) Etat(s) contractant(s) suivant(s): ES)

1. Procédé de transformation d'un hôte procaryote ou eucaryote, qui comprend le transfert dans celui-ci d'un vecteur de transfert d'ADN comprenant de l'ADN codant pour une toxine de Bacillus thuringiensis ayant la séquence d'acides aminés présentée dans SEQ ID No. 1

2. Procédé selon la revendication 1, dans lequel l'ADN a la séquence d'acides aminés présentée dans SEQ ID No. 1.

3. Procédé de lutte contre un lépidoptère nuisible, qui comprend la mise en contact du nuisible ou de son environnement avec Bacillus thuringiesis PS81RR1, disponible sous le numéro d'accès NRRL B-18458.

4. Procédé de lutte contre un lépidoptère nuisible, qui comprend la mise en contact du nuisible ou de son environnement avec un micro-organisme hôte procaryote ou eucaryote, dans lequel un vecteur de transfert d'ADN tel que défini dans la revendication ou la revendication 2 a été transféré et répliqué.

5. Procédé de lutte contre un lépidoptère nuisible, qui comprend la mise en contact du nuisible ou de son environnement avec un micro-organisme capable d'exprimer une toxine de Bacillus thuringiensis ayant la séquence d'acides aminés présentée dans la SEQ ID No. 1.

6. Procédé selon la revendication 5, dans lequel le micro-organisme est une espèce de Pseudomonas, d'Azotobacter, d'Erwinia, de Serratia de Klebsiella, de Rhizobium de Rhodopseudomonas, de Méthylophilius, d'Agrobacterium, d'Acetobacter ou d'Alcaligenes; procaryote choisi parmi les entérobactériacées, les bacillacées, les rhizobiacées, les spirillacées, les lactobacillacées, les pseudomonadacées, les azotobactéracées et les nitrobactéracées ; ou eucaryote inférieur choisi parmi les phycomycètes, les ascomycètes et les basidiomycètes.

7. Procédé selon la revendication 6, dans lequel le micro-organisme est Pseudomonas fluorescens ou Escherichia coli.

8. Procédé selon la revendication 7, dans lequel le micro-organisme est E. coli (NM522) (pMYC 390), disponible sous le numéro d'accès NRRL B-18449.

9. Procédé selon la revendication 5, dans lequel le micro-organisme est une bactérie, une levure ou un champignon pigmenté.

10. Procédé selon la revendication 7, dans lequel le micro-organisme est pigmenté et adhère au phylloplan.

11. Procédé selon l'une quelconque des revendications 3 à 10, dans lequel l'administration s'effectue sur la rhizosphère, le phylloplan ou un volume d'eau.

12. Procédé selon l'une quelconque des revendications 3 à 10, qui comprend le fait de placer un granule appât contenant le micro-organisme, par exemple sous forme de spores ou de cristaux, sur ou dans le sol au moment du semis de la graine d'une plante sur laquelle on sait que le nuisible se nourrit.

13. Procédé selon la revendication 12, dans lequel le granule appât est placé au moment même où le grain de céréale est semé dans le sol.

14. Procédé de production d'une composition comprenant un micro-organisme selon l'une quelconque des revendications 3 à 10, qui comprend la formulation du micro-organisme un véhicule pour insecticide ou avec des ingrédients de formulation, à appliquer sous forme de revêtement de semence.

15. Procédé selon la revendication 14, dans laquelle le micro-organisme est sous forme de spores ou de cristaux.

16. Procédé selon la revendication 14 ou la revendication 15, dans laquelle le véhicule comprend des phagostimulants ou des attractifs pour coléoptères.
